# EUROPEAN PATENT APPLICATION

(11) **EP 0 566 268 A1**
(43) Date of publication of application: **20.10.1993**
(21) Application number: 93302388.9
(22) Date of filing: 26.03.1993
(51) Int. Cl.: C07B 39/00, C07C 51/363, C07C 253/30

(54) **Process for the preparation of 3- or 5-fluoroaromatic compounds**

(30) Priority: 13.04.1992 GB 9208123
(71) Applicant: ZENECA LIMITED, London W1Y 6LN (GB)
(72) Inventor: Moilliet, John Stewart, Ramsbottom, Lancashire BL0 9TH (GB); Chambers, Richard Dickinson, Whitesmocks, Durham DH1 4LN (GB); Skinner, Christopher John, Turnford, Hertfordshire, EN10 6LR (GB); Atherton, Malcolm John, Preston, Lancashire PR4 0X5 (GB)
(74) Representative: Mayall, John

(57) **Abstract**

A process for the preparation of a 3- or 5-fluoroaromatic compound by reacting an aromatic compound which contains an electron-withdrawing group in the 1-position and electron-donating groups in the 2- and 4-positions with from 1% to 50% fluorine in an inert gas.

The process offers a convenient synthetic route to fluoro compounds which have substitution pattern which are difficult to obtain by other routes. The derived fluoroaromatic compounds are useful as intermediates in the manufacture of agrochemicals, pharmaceuticals and dyestuffs.

## Description

This invention relates to a process for fluorinating difluoroaromatic compounds.

Trihaloaromatic compounds are difficult to make particularly where specific substitution patterns are required for example 2,4,5-trifluoro and 2,3,4-trifluorobenzoic acids may be made by known complex, multi-stage processes which give low overall yield of desired products. The preparation of 2,4,5-trifluoro benzoic acid is described in JP 01160944 and involves chlorination of 1,2-dicyanobenzene, halogen exchange and decarboxylation reactions as part of a five stage process. A further process is described in Synthesis Letters (1990),609 and involves chlorination of phthalic anhydride, selective dechlorination, halogen exchange and decarboxylation as part of a six stage process.

We have now found a process whereby a fluoro substituent may be introduced directly substantially in a position adjacent an electron donating group in an aromatic ring of readily available substrates.

According to the present invention there is provided a process for the preparation of a 3- or 5-fluoroaromatic compound by reacting an aromatic compound which contains an electron-withdrawing group in the 1-position and electron-donating groups in the 2- and 4-positions with from 1% to 50% fluorine in an inert gas.

It is preferred that the 3- or 5-fluoroaromatic compound is of Formula (1): 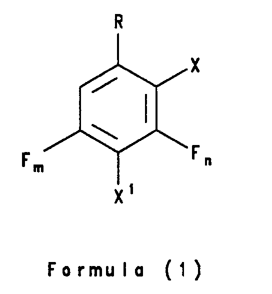
wherein:
- R: is an electron-withdrawing group;
- X and X¹: are electron-donating groups;
- n: is 0 or 1; and
- m: is 0 or 1;
provided that when n is 0, m is 1 and when n is 1, m is 0 and provided that n and m are not both 0.

The electron withdrawing group represented by R is preferably selected from -CY₃, -SO₂Y¹, -COY² and -CN in which
- Y: is selected from -F and -Cl;
- Y¹: is selected from -F, -Cl, -Br, -NH₂, -NH(C₁₋₄-alkyl) and N(C₁₋₄-alkyl)₂; and
- Y²: is selected from -H, -F, -Cl, -Br, -C₁₋₄-alkyl, -OH and -OC₁₋₄-alkyl. Especially preferred electron-withdrawing groups represented by R are -COOH and -CN.

The electron donating group may donate electrons via an inductive or a mesomeric effect or by a combination of the two effects.

The electron donating groups represented by X and X¹ may be the same or different and are preferably halogen, more preferably -F or -Cl, and especially -F.

Any of the groups represented by R may themselves undergo fluorination during the process.

It is preferred that the aromatic compound which carries an electron-withdrawing group in the 1-position and electron-donating groups in the 2- and 4-positions is of Formula (2) 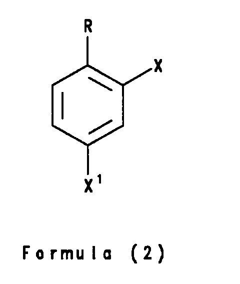
wherein: R, X, and X¹ are as hereinbefore defined.

The process is preferably performed by reacting the aromatic compound of Formula (2) neat or in a suitable reaction medium with fluorine gas. The fluorine gas is preferably diluted before use. It is preferred that the fluorine gas is diluted with an inert gas such as nitrogen. The concentration of fluorine in inert gas is preferably from 1% to 50% by volume, more preferably from 2% to 30% and especially preferably from 5% to 15%.

A suitable reaction medium is any liquid which does not react with the aromatic compound of Formula (2) or with the fluorine gas. A preferred reaction medium is a liquid such as a perhaloalkane for example carbon tetrachloride and 1,1,2-trichloro-1,2,2-trifluoro ethane, or a perhaloacetic acid for example trifluoroacetic acid or trichloroacetic acid, or acetonitrile or a mixture thereof. The reaction medium is preferably acetonitrile or trifluoroacetic acid.

The process may be carried out at a temperature from -40°C to 25°C, preferably from -30°C to 20°C. It is preferred that the process in a liquid such as acetonitrile is carried out at a temperature from -35°C to -5°C and more preferably at temperatures from -30°C to -15°C. It is preferred that the process in a liquid such as trifluoroacetic acid is carried out at a temperature from -5°C to 25°C, more preferably at a temperature from 0°C to 20°C, and especially preferably at a temperature from 5°C to 20°C. Reactions in acetonitrile are at lower temperature than those in trifluoroacetic acid to minimise reaction of acetonitrile with the fluorine. Although the ratio of fluorine to the aromatic compound of Formula (2) may be varied within wide limits, it is preferred that the molar ratio of fluorine to aromatic compound of Formula (2) is from 1:1 to 2:1 in order to promote a good yield of the desired products and inhibit the formation of overfluorinated by-products.

When the fluorination reaction is substantially complete the product may be isolated in any convenient manner. For example the reaction mixture may be purged with nitrogen to remove residual fluorine and the reaction medium removed by distillation. Residual product may be purified by any convenient means such as fractional distillation at atmospheric pressure or under reduced pressure or alternatively by recrystallisation from a suitable solvent if the product is a solid.

Separation of isomers may be achieved by chromatography, or if R is -COOH by separation at different pH values or if R is -COOalkyl by fractional distillation.

The 3- or 5-fluoroaromatic compounds of Formula (1) are useful as synthetic intermediates for example in the manufacture of agrochemicals, pharmaceuticals, such as quinolone antibacterials and dyestuffs.

The present process offers a convenient synthetic route to fluoro compounds which have substitution patterns which are difficult to achieve by other routes.

Furthermore, the 3- or 5-fluoroaromatic compounds of Formula (1) may be readily converted into 2-fluoro-1,3-dihalo or 1-fluoro-2,4-dihalo compounds by removal of the group R by methods known in the art.

The invention is illustrated by the following examples.

### Example 1

A mixture of 2,4-difluorobenzoic acid (0.01 mol) in trifluoroacetic acid (15 parts) was cooled to 15°C under a flow of nitrogen. A 10% by volume mixture of fluorine (0.02 mol) in nitrogen was passed through the cooled solution at 15°C. When all the fluorine had been added the reaction mixture was warmed to ambient temperature under a flow of nitrogen. The reaction mixture was analysed by 19F nmr and mass spectrometry using authentic samples as standards. From the analysis >95% of the 2,4-difluorobenzoic acid was converted to fluorinated product, the fluorinated product comprised 66% 2,4,5-trifluorobenzoic acid and 33% 2,3,4-trifluorobenzoic acid.

### Example 2

A mixture of 2,4-difluorobenzonitrile (0.015 mol) in acetonitrile (15 parts) was cooled to -30°C under a flow of nitrogen. A 10% by volume mixture of fluorine (0.03 mol) in nitrogen was passed through the cooled solution at -30°C. When all the fluorine had been added the reaction mixture was warmed to ambient temperature under a flow of nitrogen. The reaction mixture was analysed as for Example 1. From the analysis 65% of the 2,4-difluorobenzonitrile was converted to fluorinated product, the fluorinated product comprised 69% 2,4,5-trifluorobenzonitrile and 23% 2,3,4-trifluorobenzonitrile and 8% compounds containing a trifluoromethyl group.

## Claims

1. A process for the preparation of a 3- or 5-fluoroaromatic compound by reacting an aromatic compound which contains an electron-withdrawing group in the 1-position and electron-donating groups in the 2- and 4-positions with from 1% to 50% fluorine in an inert gas.

2. A process according to Claim 1 wherein the aromatic compound which contains an electron-withdrawing group in the 1-position and electro-donating groups in the 2- and 4-positions is of Formula (2): 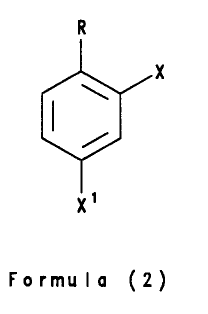 wherein R is -CY₃, -SO₂Y¹, -COY² or -CN;
in which
Y is -F or -Cl;
Y¹ is -F, -Cl, -Br, -NH₂, -NH(C₁₋₄-alkyl) or -N(C₁₋₄-alkyl)₂; and
Y² is -H, -F, -Cl, -Br, -C₁₋₄-alkyl, -OH or -OC₁₋₄-alkyl; and X and X¹ are each independently halogen.

3. A process according to Claim 2 wherein in the compound of Formula (2)
R is -COOH or -CN
X and X¹ are each independently -F or -Cl.

4. A process according to any one of Claims 1 to 3 in which the aromatic compound in a liquid medium is reacted with the fluorine gas.

5. A process according to Claim 4 wherein the liquid medium is a perhaloalkane or a perhaloacetic acid or acetonitrile or a mixture thereof.

6. A process according to Claim 4 wherein the liquid medium is carbon tetrachloride, 1,1,2-trichloro-1,2,2,-trifluoroethane, trifluoroacetic acid, trichloroacetic acid or a acetonitrile or a mixture thereof.

7. A process according to any one of Claims 1 to 6 carried out at a temperature of from -40°C to 25°C.

8. A process according to any one of Claims 1 to 7 wherein the molar ratio of fluorine to aromatic compound is from 1:1 to 2:1.
